(19) Europäisches Patentamt
European Patent Office
Office européen des brevets

(11) Veröffentlichungsnummer: **0 191 734**
**B1**

(12) # EUROPÄISCHE PATENTSCHRIFT

(45) Veröffentlichungstag der Patentschrift: **10.01.90**

(51) Int. Cl.⁴: **C 07 D 275/06**, A 01 N 43/80

(21) Anmeldenummer: **86810067.8**

(22) Anmeldetag: **07.02.86**

(54) **Schädlingsbekämpfungsmittel.**

(30) Priorität: **13.02.85 CH 649/85**

(43) Veröffentlichungstag der Anmeldung: **20.08.86 Patentblatt 86/34**

(45) Bekanntmachung des Hinweises auf die Patenterteilung: **10.01.90 Patentblatt 90/2**

(84) Benannte Vertragsstaaten: **AT BE CH DE FR GB IT LI NL**

(56) Entgegenhaltungen: **EP-A- 0 110 829**

(73) Patentinhaber: **CIBA-GEIGY AG, Klybeckstrasse 141, CH-4002 Basel (CH)**

(72) Erfinder: **Drabek, Jozef, Dr., Benkenstrasse 12, CH-4104 Oberwil (CH)**

**EP 0 191 734 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues 3-Acylaminobenzisothiazol-S,S-dioxid, das zur Bekämpfung von Insekten geeignet ist, Verfahren zu seiner Herstellung und insektizide Mittel, die diese Verbindung als aktive Komponente enthalten.

Das erfindungsgemäss vorgeschlagene 3-Acetylamino-4-methoxy-benzisothiazol-S,S-dioxid hat die Formel I

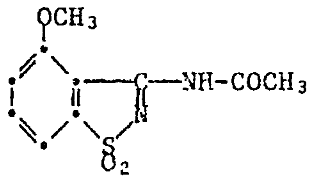

(I)

Die Verbindung der Formel I kann im Rahmen der vorliegenden Erfindung auch in ihrer tautomeren Form der folgenden Formel Ia

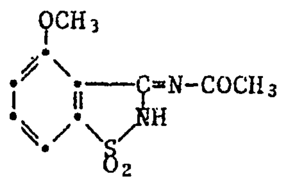

(Ia)

vorliegen.

Aus der europäischen Patentanmeldung Nr. 0 033 984 sind bereits substituierte 3-Aminobenzisothiazol-S,S-dioxide mit aphizider Wirkung bekannt. Ferner werden in der veröffentlichten europäischen Patentanmeldung Nr. 0 110 829 3-Acylaminobenzisothiazol-S,S-dioxide und deren Verwendung als Insektizide beschrieben, wobei die erfindungsgemässe Verbindung der Formel I von dem dort angegebenen allgemeinen Umfang umfasst, aber als solche nicht offenbart wird. Es hat sich überraschenderweise herausgestellt, dass die vorliegende Verbindung der Formel I den aus der erwähnten europäischen Patentanmeldung bekannten strukturähnlichen Verbindungen hinsichtlich ihrer insektiziden, insbesondere aphiziden, Wirkung überlegen ist (vgl. das nachfolgende Beispiel 4). Es war nicht hervorzusehen, dass sich die erfindungsgemässe Verbindung der Formel I, deren Herstellung aus relativ leicht zugänglichen Ausgangsstoffen möglich ist, selbst bei geringen Aufwandmengen zur vollständigen Vertilgung von Populationen saugender Schadinsekten eignet, wobei auch die geringe Warmblütertoxizität, die gute Pflanzenverträglichkeit, die günstige Dauerwirkung und die vorteilhafte Formulierbarkeit der neuen Verbindung zu erwähnen ist.

Die erfindungsgemässe Verbindung der formel I kann analog bekannten Verfahren hergestellt werden (vgl. z.B. J. March, «Advanced Organic Chemistry»; McGraw Hill, New York, 1977, s. 383-385), indem man

a.) das 3-Amino-4-methoxy-benzisothiazol-S,S-dioxid der Formel II

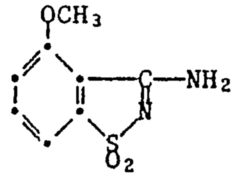

(II)

mit einem Acetylhalogenid der Formel III

X-$COCH_3$ (III)

oder mit Acetanhydrid gegebenenfalls in Gegenwart einer Base, umsetzt, wobei in Formel III X Halogen, vorzugsweise Chlor, bedeutet; oder

b.) das 3-Acetylamino-4-methoxy-benzisothiazol der Formel IV

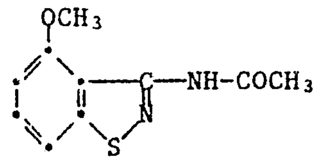

(IV)

in an sich bekannter Weise oxidiert.

Als geeignete Basen kommen für das vorliegende Verfahren a.) insbesondere tertiäre Amine, wie Trialkylamine, Dialkylaniline und p-Dialkylaminopyridine, in Betracht. Das Verfahren wird im allgemeinen bei normalem Druck, bei einer Temperatur von –25°C bis 150°C, vorzugsweise 50° bis 100°C, und gegebenenfalls in einem Lösungs- oder Verdünnungsmittel durchgeführt.

Als Oxidationsmittel für das vorliegende Verfahren b.) können Persäuren, z.B. Perameisensäure, Peressigsäure, substituierte Perbenzoesäuren, Natriumperjodat, Monopermaleinsäure, Monoperphthalsäure, tert.-Butylperbenzoesäure, Trifluorperessigsäure oder Wasserstoffperoxid, insbesondere Perbenzoesäure, verwendet werden. Die Oxidation wird im allgemeinen bei einer Reaktionstemperatur zwischen –30° und 120°C, meist zwischen 0° und 50°C, bei normalem oder erhöhtem Druck und vorzugsweise in einem inerten Lösungs- oder Verdünnungsmittel durchgeführt.

Für die erfindungsgemässen Verfahren eignen sich als Lösungs- oder Verdünnungsmittel z.B. Ether und etherartige Verbindungen, wie Diethylether, Dipropylether, Dioxan, Dimethylethan und Tetrahydrofuran; Amide, wie N,N-dialkylierte Carbonsäureamide; aliphatische, aromatische oder halogenierte Kohlenwasserstoffe, insbesondere Benzol, Toluol, Xylole, Chloroform und Chlorbenzol; Nitrile, wie Acetonitril; Dimethylsulfoxid und Ketone, wie Aceton und Methylethylketon, sowie Ester, z.B. Essigsäureethylester.

Der Ausgangsstoff der Formel II ist bekannt und kann nach bekannten Verfahren hergestellt werden. So wird die Herstellung von 3-Aminobenzisothiazol-S,S-dioxiden in der europäischen Patentanmeldung Nr. 0 033 984 beschrieben. Die Ausgangsverbindung der Formel IV ist ebenfalls bekannt und kann

erhalten werden durch Acetylierung des 3-Amino-4--methoxy-benzisothiazols.

Die Verbindung der Formel I ist zur Bekämpfung von Insekten an Tieren und Pflanzen geeignet.

Insbesondere eignet sich die Verbindung der Formel I zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Vor allem eignet sich die Verbindung der Formel I zur Bekämpfung von pflanzenschädigenden Insekten, speziell pflanzenschädigenden Insekten in Zier- und Nutzpflanzungen, insbesondere in Baumwollkulturen, Gemüsekulturen, Reiskulturen und Obstkulturen. In diesem Zusammehang ist hervorzuheben, dass die genannte Verbindung sich durch eine stark ausgeprägte systemische aber auch Kontakt-Wirkung gegen saugende Insekten, vor allem gegen Insekten der Familie Aphididae (wie z.B. Aphis fabae, Aphis craccivora und Myzus persicae), welche sich mit bekannten Mitteln nur schwierig bekämpfen lassen, auszeichnet. Sie zeigt auch eine günstige Wirkung gegen fressende und beissende Insekten sowie gegen Fliegen, wie z.B. Musca domestica, und Mückenlarven.

Die Wirkung der erfindungsgemässen Verbindung bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht: organische Phosphorverbindungen, Nitrophenole und Derivate, Formamidine, Harnstoffe, Carbamate, chlorierte Kohlenwasserstoffe, Pyrethroide und Bacillus thuringiensis-Präparate.

Mit besonderem Vorteil kann man die Verbindung der Formel I auch mit Substanzen kombinieren, welche einen pestizid verstärkenden Effekt ausüben. Beispiele solcher Verbindungen sind u.a.: Piperonylbutoxid, Propinylether, Propinyloxime, Propinylcarbamate und Propinylphosphonate, 2-(3,4-Methylendioxyphenoxy)-3,6,9-trioxaundecan oder S,S,S-Tributylphosphortrithioat.

Die gute insektizide Wirkung der erfindungsgemäss vorgeschlagenen Verbindung der Formel I entspricht einer Abtötungsrate (Mortalität) von mindestens 50-60% der erwähnten Schadinsekten.

Die Verbindung der Formel I wird in unveränderter Form oder vorzugsweise zusammen mit den in der Formulierungstechnik üblichen Hilfsmitteln eingesetzt und wird daher z.B. zu Emulsionen, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den erfindungsgemässen Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen des Wirkstoffs mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien, z.B. Calcit oder Sand in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierte Ammoniumsalze von höheren Fettsäuren ($C_{10}$-$C_{22}$), wie z.B. die Na- oder K-Salze der Öl- oder Stearinsäure, oder Salze von natürlichen Fettsäuregemischen, die z.B. aus Kokosnuss- oder Tallöl gewonnen werden können. Ferner sind als Tenside auch die Fettsäuremethyltaurinsalze sowie modifizierte und nicht modifizierte Phospholipide zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und

Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethylaminsalze der Dodecylbenzolsulfonsäure, der Dibutylnaphathalinsulfonsäure, oder eines Naphthalinsulfonsäure-Formaldehydkondensationsproduktes.

Ferner kommen auch entsprechende Phosphate, wie z.B. Salze des Phosphorsäureesters eines p-Nonylphenol-(4-14)-Ethylenoxid-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Ethylenglykoleinheiten.

Als Beispiele nichtionischer Tenside seien Nonylphenolpolyethoxyethanole, Ricinusölpolyglykolether, Polypropylenpolyethylenoxid-Addukte, Tributylphenoxypolyethoxyethanol, Polyethylenglykol und Octylphenoxypolyethoxyethanol erwähnt.

Ferner kommen auch Fettsäureester von Polyoxyethylensorbitan, wie das Polyoxyethylensorbitantrioleat, in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:

«Mc Cutcheon's Detergents and Emulsifiers Annual» MC Publishing Corp., Ridgewood, New Jersey, 1979;

Dr. Helmut Stache «Tensid Taschenbuch», Carl Hanser Verlag, München/Wien 1981.

Die pestiziden Zubereitungen enthalten in der Regel 0,1 bis 99%, insbesondere 0,1 bis 95%, Wirkstoff der Formel I, 1 bis 99% eines festen oder flüssigen Zusatzstoffes und 0 bis 25%, insbesondere 0,1 bis 25%, eines Tensides. Während als Handelsware eher konzentrierte Mittel bevorzugt werden, verwendet der Endverbraucher in der Regel verdünnte Mittel, die geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

*Beispiel 1*

*Herstellung von 3-Acetylamino-4-methoxy-benzisothiazol-S,S-dioxid*

Es werden 6,66 g 3-Acetylamino-4-methoxybenzisothiazol mit 150 ml Chloroform vermischt. Zu diesem Gemisch werden unter Rühren bei einer Temperatur von 20 bis 30°C portionsweise 10,35 g m-Chlorperbenzoesäure zugegeben. Das Reaktionsgemisch wird danach über Nacht bei Raumtemperatur gerührt, sodann abgenutscht und aus dem Filtrat das Lösungsmittel abdestilliert. Das als Rückstand erhaltene Rohprodukt wird chromatographiert (Kieselgel-Säule; Elutionsmittel: Methylenchlorid-Isopropanol). Auf diese Weise erhält man die Titelverbindung als farblose Kristalle vom Schmelzpunkt 242-245°C.

*Beispiel 2*

*Formulierungen für den Wirkstoff der Formel I gemäss Beispiel 1 resp. Kombinationen dieses wirkstoffes mit andern Insektiziden* (% = Gewichtsprozent)

| 2.1 *Spritzpulver* | *a)* | *b)* | *c)* |
|---|---|---|---|
| Wirkstoff oder Wirkstoffkombination | 25% | 50% | 75% |
| Na-Ligninsulfonat | 5% | 5% | — |
| Na-Laurylsulfat | 3% | — | 5% |
| Na-Diisobutylnaphthalinsulfonat | — | 6% | 10% |
| Octylphenolpolyethylenglykolether (7-8 Mol AeO) | — | 2% | — |
| Hochdisperse Kieselsäure | 5% | 10% | 10% |
| Kaolin | 62% | 27% | — |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.2 *Emulsions-Konzentrat* | |
|---|---|
| Wirkstoff oder Wirkstoffkombination | 10% |
| Octylphenolpolyethylenglykolether (4-5 Mol AeO) | 3% |
| Ca-Dodecylbenzolsulfonat | 3% |
| Ricinusölpolyglykolether (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 2.3 *Stäubemittel* | *a)* | *b)* |
|---|---|---|
| Wirkstoff oder Wirkstoffkombination | 5% | 8% |
| Talkum | 95% | — |
| Kaolin | — | 92% |

Man erhält anwendungsfertige Stäubemittel, indem der Wirkstoff oder die Wirkstoffkombination mit dem Träger vermischt und auf einer geeigneten Mühle vermahlen wird.

2.4 *Extruder-Granulat*

| | |
|---|---|
| Wirkstoff oder Wirkstoffkombination | 10% |
| Na-Ligninsulfonat | 2% |
| Carboxymethylcellulose | 1% |
| Kaolin | 87% |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

2.5 *Umhüllungs-Granulat*

| | |
|---|---|
| Wirkstoff oder Wirkstoffkombination | 3% |
| Polyethylenglykol | 3% |
| Kaolin | 94% |

Der fein gemahlene Wirkstoff oder die Wirkstoffkombination wird in einem Mischer auf das mit Polyethylenglykol angefeuchtete Kaolin gleichmässig aufgetragen. Auf diese Weise erhält man staubfreie Umhüllungs-Granulate.

2.6 *Suspensions-Konzentrat*

| | |
|---|---|
| Wirkstoff oder Wirkstoffkombination | 40 % |
| Ethylenglykol | 10 % |
| Nonylphenolpolyethylenglykolether (15 Mol AeO) | 6 % |
| Na-Ligninsulfonat | 10 % |
| Carboxymethylcellulose | 1 % |
| 37%ige wässrige Formaldehyd-Lösung | 0,2% |
| Silikonöl in Form einer 75%igen wässrigen Emulsion | 0,8% |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

*Beispiel 3*

*Insektizide Kontaktwirkung gegen Aphis craccivora*

In Töpfen angezogene Bohnen-Pflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 100 ppm der zu prüfenden Verbindung bis zur Tropfnässe besprüht. Man verwendet für die zu prüfende Verbindung zwei Pflanzen; die Auswertung der erzielten Abtötungsrate erfolgt nach weiteren 24 Stunden.

Die erfindungsgemässe Verbindung der Formel I zeigt 90-100% Wirkung (Mortalität) im obigen Test.

*Beispiel 4*

*Insektizide Kontaktwirkung gegen Myzus persicae*

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 400 ppm der zu prüfenden Verbindung bis zur Tropfnässe besprüht. Eine Auswertung der erzielten Abtötungsrate erfolgt 48 Stunden nach Applikation. Der Versuch wird bei 20-22°C und 60% relativer Luftfeuchtigkeit durchgeführt.

Die erfindungsgemässe Verbindung der Formel I zeigt 90-100% Wirkung (Moralität) im obigen Test.

*Beispiel 5*

*Insektizide systemische Wirkung gegen Myzus persicae (Erde)*

Bewurzelte Kohlpflanzen im 4- bis 5-Blatt-Stadium werden in Töpfe, welche 60 ccm Erde enthalten, verpflanzt. Anschliessend werden 50 ml einer wässrigen Formulierung der zu prüfenden Verbindung I (erhalten aus einem 25%igen Spritzpulver) in einer Konzentration von 12,5 ppm direkt auf die Erde aufgegossen, ohne die Pflanzen selbst zu benetzen.

Nach 24 Stunden werden auf die oberirdischen Pflanzenteile der behandelten Pflanzen Blattläuse der Spezies Myzus persicae gesetzt und die Pflanzen mit Plastikzylindern abgedeckt, um die Blattläuse von einer eventuellen Kontakt- oder Gaswirkung der Testsubstanz zu schützen.

Die Auswertung der erzielten %-Abtötung erfolgt 7 Tage nach Versuchsbeginn. Für die Testsubstanz werden zwei Pflanzen, je eine in separaten Töpfen, verwendet. Der Versuch wird bei ca. 25°C und 60% relativer Luftfeuchtigkeit durchgeführt.

Die erfindungsgemässe Verbindung der Formel I zeigt 90-100% Wirkung (Mortalität) im obigen Test.

*Beispiel 6*

*Insektizide systemische Wirkung gegen Aphis craccivora und Myzus persicae*

Erbsenkeimlinge, die 24 Stunden vor Beginn des Versuches mit den Blattläusen infestiert worden waren, werden in 20 ml einer wässrigen Brühe gestellt, die 3 bzw. 0,75 ppm des zu prüfenden Wirkstoffes enthält. Die wässrige Brühe wird aus einem Emulsionskonzentrat oder einer benetzbaren Pulverzubereitung des betreffenden Wirkstoffes hergestellt und befindet sich in einem Gefäss, das mit einem Löcher aufweisenden Plastikdeckel abgeschlossen ist. Die Wurzel der infestierten Erbsenpflanze wird jeweils durch ein Loch in dem Plastikdeckel in die Brühe geschoben. Das Loch wird dann mit Watte abgedichtet, um die Pflanze zu fixieren und den Einfluss der Gasphase aus der Brühe auszuschalten.

Der Versuch wird bei 20°C und 60% relativer Luftfeuchtigkeit durchgeführt. Nach zwei Tagen wird auf die Anzahl der nicht mehr saugfähigen Testtiere, im Vergleich zu unbehandelten Kontrollen bonitiert. Damit wird festgestellt, ob der über die Wurzel aufgenommene Wirkstoff die Blattläuse an den oberen Pflanzenteilen abtötet.

In der folgenden Tabelle I sind Versuchsergebnisse auf der Basis des obigen Beispiels aufgeführt.

Tabelle I

| verwendete Verbindung | Systemische Wirkung – % Mortalität | | | | | |
|---|---|---|---|---|---|---|
| | Aphis craccivora | | Myzus persicae – nicht resistent | | Myzus persicae – gegen OP-Verbindungen und Carbamate resistent | |
| | Wirkstoffkonzentration | | | | | |
| | 3 ppm | 0,75 ppm | 3 ppm | 0,75 ppm | 3 ppm | 0,75 ppm |
| Verbindung der Formel I gemäss vorliegender Erfindung 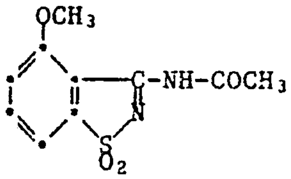 | 100% | 100% | 100% | 95% | 100% | 100% |
| Verbindung gemäss EP-Patentanm. Nr. 0 110 829 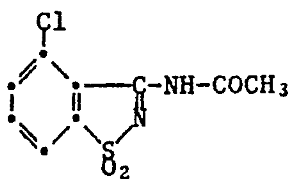 | 100% | 0% | 95% | 0% | 90% | 0% |

*Beispiel 7*

*Insektizide Blattpenetrations-Wirkung gegen Aphis craccivora*

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der becher wird mit einem Kunststoffdeckel, der in der Mitte eine ausgestanzte Öffnung von 2 cm Durchmesser aufweist, bedeckt. Auf die in dem Deckel befindliche Öffnung wird ein Blatt einer Vicia faba-Pflanze gelegt, ohne dieses Blatt von der eingetropften Pflanze abzutrennen. Das Blatt wird dann mit einem zweiten Lochdeckel auf dem Becher über der Öffnung des Deckels fixiert. Von der Unterseite her, d.h. durch die Öffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zur prüfenden Wirkstoffes in einer Konzentration von 100 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende Blattläuse abzutöten.

Der Versuch wird bei etwa 20°C und 60% relativer Luftfeuchtigkeit durchgeführt. Die Auswertung auf %-Mortalität erfolgt 48 Stunden nach Wirkstoffapplikation.

Die erfindungsgemässe Verbindung der Formel I zeigt 90-100% Wirkung (Mortalität) im obigen Test.

**Patentansprüche**

1. Verbindung der Formel I

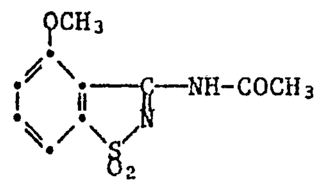 (I)

einschliesslich ihrer tautomeren Form.

2. Verfahren zur Herstellung der Verbindung der Formel I gemäss Anspruch 1, dadurch gekennzeichnet, dass man

a) die Verbindung der Formel II

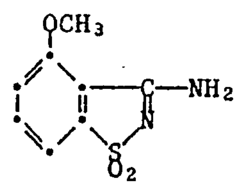 (II)

mit einer Verbindung der Formel III

$$X\text{-}COCH_3 \qquad (III)$$

oder mit Acetanhydrid umsetzt, wobei in Formel III X Halogen, vorzugsweise Chlor, bedeutet; oder

b) die Verbindung der Formel IV

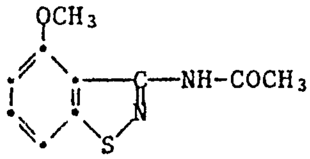

(IV)

oxidiert.

3. Schädlingsbekämpfungsmittel, welches als aktive Komponente die Verbindung der Formel I gemäss Anspruch 1, zusammen mit geeigneten Trägern und/oder anderen Zuschlagstoffen enthält.

4. Verwendung der Verbindung der Formel I gemäss Anspruch 1 zur Bekämpfung on Insekten.

5. Verwendung gemäss Anspruch 4 zur Bekämpfung pflanzenschädigender Insekten.

6. Verwendung gemäss Anspruch 5 zur Bekämpfung pflanzenschädigender saugender Insekten.

7. Verwendung gemäss Anspruch 6 zur Bekämpfung von Insekten der Familie Aphididae.

8. Verfahren zur Bekämpfung von Insekten, dadurch gekennzeichnet, dass man die Insekten bzw. deren verschiedene Entwicklungsstadien oder deren Aufenthaltsort mit einer pestizid wirksamen Menge der Verbindung der Formel I gemäss Anspruch 1 oder mit einem Mittel enthaltend neben Zusatz- und Trägerstoffen eine pestizid wirksame Menge dieser Verbindung, in Kontakt bringt oder behandelt.

**Claims**

1. The compound of formula I

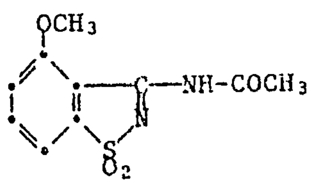

(I)

or the tautomer thereof.

2. A process for the preparation of the compound of formula I according to claim 1, which comprises

a) reacting the compound of formula II

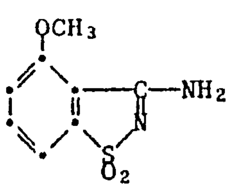

(II)

with a compound of formula III

X-$COCH_3$ (III)

or with acetic anhydride, where X in formula III is halogen, preferably chlorine; or

b) oxidising the compound of formula IV

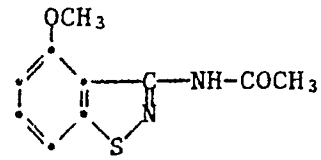

(IV)

3. A pesticidal composition which contains as active component the compound of formula I according to claim 1, together with suitable carriers and/or other adjuvants.

4. Use of the compound of formula I according to claim 1 for controlling insects.

5. Use according to claim 4 for controlling plant-destructive insects.

6. Use according to claim 5 for controlling plant-destructive sucking insects.

7. Use according to claim 6 for controlling insects of the family Aphididae.

8. A method of controlling insects, which comprises contacting or treating the insects and their various development stages, or the locus thereof, with a pesticidally effective amount of the compound of formula I according to claim 1 or with a composition containing a pesticidally effective amount of the compound together with adjuvants and carriers.

**Revendications**

1. Le composé de formule I

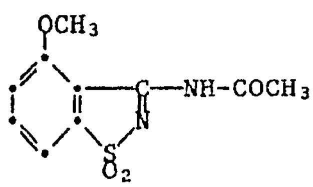

(I)

y compris sa forme tautomère.

2. Procédé de préparation du composé de formule I selon la revendication 1, caractérisé en ce que

a) on fait réagir le composé de formule II

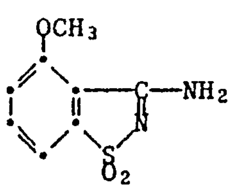

(II)

avec un composé de formule III

X-$COCH_3$ (III)

ou avec l'anhydride acétique, le symbole X de la formule III représentant un halogène, de préférence le chlore, ou bien

b) on oxyde le composé de formule IV

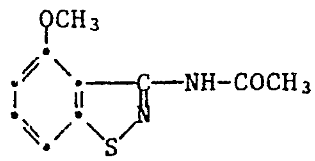

(IV)

3. Produit pesticide contenant en tant que composant actif le composé de formule I de la revendication 1 avec des véhicules et/ou d'autres additifs appropriés.

4. Utilisation du composé de formule I selon la revendication 1 pour la lutte contre les insectes.

5. Utilisation selon la revendication 4 pour la lutte contre les insectes nuisibles pour les végétaux.

6. Utilisation selon la revendication 5 pour la lutte contre les insectes suceurs nuisibles pour les végétaux.

7. Utilisation selon la revendication 6 pour la lutte contre les insectes de la famille des Aphididae.

8. Procédé pour combattre les insectes, caractérisé en ce que l'on met en contact ou on traite les insectes ou leurs divers stades de développement ou leur biotope par une quantité pesticide efficace du composé de formule I selon la revendication 1 ou par un produit contenant, avec des additifs et véhicules, une quantité pesticide efficace de ce composé.